Europäisches Patentamt

European Patent Office

Office européen des brevets

(11) Publication number: **0 418 657 A2**

## (12) EUROPEAN PATENT APPLICATION

(21) Application number: 90117103.3

(22) Date of filing: 05.09.90

(51) Int. Cl.5: **C07C 57/05, C07C 47/22**

(30) Priority: 18.09.89 JP 242945/89

(43) Date of publication of application:
27.03.91 Bulletin 91/13

(84) Designated Contracting States:
BE DE ES FR GB IT NL

(71) Applicant: SUMITOMO CHEMICAL COMPANY, LIMITED
5-33, Kitahama 4-chome Chuo-ku
Osaka(JP)

(72) Inventor: Nagai, Koichi
4-13, Tsuchihashi-2-chome
Niihama-shi(JP)
Inventor: Nagaoka, Yoshihiko
3-746, Ikkucho-2-chome
Niihama-shi(JP)
Inventor: Sato, Hiroshi
4-13, Hoshigoecho
Niihama-shi(JP)
Inventor: Ohsu, Motomasa
6-3-104, Honmachi-9-chome
Toyonaka-shi(JP)

(74) Representative: Henkel, Feiler, Hänzel & Partner
Möhlstrasse 37
W-8000 München 80(DE)

(54) Process for producing methacrylic acid and methacrolein by catalytic oxidation of isobutane.

(57) The process for producing methacrylic acid and methacrolein of the present invention comprises the step of subjecting isobutane to gas phase catalytic oxidation with molecular oxygen by the use of a catalyst comprising either a heteropolyacid salt or a mixture of a heteropolyacid and a salt thereof, said catalyst having the following composition determined for elements other than oxygen, nitrogen and hydrogen,

$P_a Mo_b X_c Y_d Z_e$

wherein P and Mo are phosphorus and molybdenum, respectively,

X is at least one member selected from the group consisting of rubidium, cesium and thallium,

Y is at lest one member selected from the group consisting of vanadium and arsenic,

Z is at least one member selected from the group consisting of copper, silver, bismuth, iron, cobalt, antimony, lanthanum and cerium,

the suffixes a, b, c, d and e indicate the atomic ratio between the elements, and

a : b : c : d : e is at most 3 : 12 : at most 3 : at most 3 : O-3.

According to said process, methacrylic acid and methacrolein can be directly obtained with high selectivity at a lower temperature than before by using a high concentration of isobutane, and moreover the catalytic lifetime is greatly improved.

# PROCESS FOR PRODUCING METHACRYLIC ACID AND METHACROLEIN BY CATALYTIC OXIDATION OF ISOBUTANE

The present invention relates to a process for producing methacrylic acid and methacrolein by oxidizing isobutane. Particularly, it relates to a process for producing methacrylic acid and methacrolein by subjecting isobutane to gas phase catalytic oxidation with molecular oxygen by the use of a specific catalyst.

A process for producing methacrylic acid from isobutylene or tertiary butanol by two-stage oxidation via methacrolein has been well known and already industrially practiced.

As processes for producing methacrylic acid by using, as starting material, isobutane which is more inexpensive than isobutylene or tertiary butanol, there are known the following processes:

(1) JP-A-58-189130 discloses a process which comprises dehydrogenating isobutane to obtain isobutylene, oxidizing isobutylene without separation and purification, thereby producing methacrolein and methacrylic acid. In this case, the same bismuthmolybdate based catalyst as in conventional processes is used in the oxidation step of isobutylene. The unreacted isobutane in the dehydrogenation step is considered to be substantially an inert gas.

(2) JP-A-55-62041 discloses a process which comprises subjecting isobutane directly to gas phase catalytic oxidation with molecular oxygen by the use of a heteropolyacid-based catalyst containing molybdenum, phosphorus and antimony, thereby obtaining methacrylic acid. According to this process, the following results are obtained. When the isobutane concentration is 10%, there are attained a conversion of 10%, a selectivity for methacrylic acid of 50% and a selectivity for methacrolein of 20%. When the isobutane concentration is 28%, there are attained a conversion of 9%, a selectivity for methacrylic acid of 46% and a selectivity for methacrolein of 10%.

(3) JP-A-62-132832 disclose a process for producing methacrylic acid and methacrolein which comprises bringing isobutane and oxygen into contact with a heteropolyacid catalyst alternately.

(4) JP-A-63-145249 discloses a process using a reduced heteropolyacid catalyst.

In the above processes (3) and (4), a conversion of isobutane of 5 to 12% and a total selectivity for methacrolein and methacrylic acid of approximately 60 - 75% are attained using various catalysts.

However, there prior art processes are not satisfactory for industrial practice in consideration of the reaction conditions, etc.

The process disclosed in JP-A-58-189130 (1) is not always advantageous because of its complexity.

In the processes of simultaneous feeding of isobutane and oxygen disclosed in JP-A-55-62041 (2) and JP-A-63-145249 (4), when the isobutane concentration is 10%, the conversion is only about 10%, that is, the productivity of methacrylic acid is too low. When the isobutane concentration is 28%, the lowering selectivities for methacrylic acid and methacrolein is observed.

The process of alternate contact of isobutane and oxygen disclosed in JP-A-62-132832 (3) seems to involve many difficult problems in its reduction to industrial practice.

In addition, in the examples given in the above references, there is employed a reaction temperature of 340° to 350° C. However, the temperature is too high for a heteropolyacid-based catalyst to be used from the viewpoint of catalytic life-time, and the examples cannot directly be reduced to practice.

The present inventor earnestly investigated an efficient process for producing methacrylic acid and methacrolein by catalytic oxidation of isobutane, and consequently found that a heteropolyacid-based catalyst having a specific composition is excellent in activity of reaction, selectivity for desired products and catalytic lifetime, whereby the present invention was accomplished.

According to the present invention, there is provided a process for producing methacrylic acid and methacrolein which comprises the step of:

subjecting isobutane to gas phase catalytic oxidation with molecular oxygen by the use of a catalyst comprising either a heteropolyacid salt or a mixture of a heteropolyacid and a salt thereof, said catalyst having the following composition determined for elements other than oxygen, nitrogen and hydrogen,

$P_a Mo_b X_c V_d Z_e$

wherein P and Mo are phosphorus and molybdenum, respectively,

X is at least one member selected from the group consisting of rubidium, cesium and thallium,

Y is at least one member selected from the group consisting of vanadium and arsenic,

Z is at least one member selected from the group consisting of copper, silver, bismuth, iron, cobalt, antimony, lanthanum and cerium,

the suffixes a, b, c, d and e indicate the atomic ratio between the elements, and

a:b:c:d:e is at most 3 : 12 : at most 3 : at most 3 : 0-3.

The catalyst used in the present invention can be prepared by various conventional methods. For example, a free heteropolyacid composed of phosphorus, molybdenum and at least one of vanadium and arsenic can be obtained by heating an aqueous slurry containing phosphoric acid, molybdenum trioxide, vanadium pentaoxide and arsenic acid for a long period of time. Phosphomolybdic acid can also be used in place of phosphoric acid and molybdenum trioxide. A catalyst having a predetermined composition can be obtained by adding at least one component X such as rubidium and optionally at least one component Z such as copper to the free heteropolyacid, in the form of an aqueous nitrate solution, followed by evaporation to dryness and calcination.

When the amount of the component X is small, the structure of the catalyst obtained by the above method is a mixture of a cubic salt structure and a free acid structure.

A catalyst obtained by preparing a composition in the same manner as described above except for adding ammonium ions or organic amine ions such as pyridinium ions together with the component X, and calcining the composition at 400° to 500°C in an inert gas to liberate the whole or a portion of ammonia or the organic amine, has a cubic salt structure. Such a method is a preparation method of a satisfactory catalyst. Although in the case of this catalyst, the heteropolyacid is in partially reduced state, the catalyst of the present invention need not necessarily to be in reduced state. A catalyst obtained by re-calcining the catalyst obtained in the manner described above, at 400°C or lower in air after the calcination in an inert gas also has an equivalent advantage. A catalyst composed of a mixture of a non-reduced heteropolyacid and a non-reduced salt thereof also gives a satisfactory result.

The catalyst used in the present invention has a heteropolyacid structure of the so-called Keggin type. It is characterized by having a crystal structure at least a part of which is a salt of the component X, i.e., rubidium, cesium or univalent thallium. Such a salt improves the heat stability of the heteropolyacid structure and increases the surface area, and therefore it can be speculated that the salt contributes to the improvement of the activity and the catalytic lifetime. Too large an amount of the component X causes a lowering of the activity and hence is not desirable.

The component Y, vanadium and/or arsenic as essential constituent has an enhancing effect on the selectivity for methacrylic acid and methacrorein. It is conjectured that in the heteropolyacid structure, vanadium atom(s) replace molybdenum atom(s), and arsenic atom(s) replace phosphorus atom(s). Vanadium alone, arsenic alone and a combination of vanadium and arsenic are equivalent in the above effect. Too large an amount of the component Y causes a lowering of the activity and hence is not desirable.

Although the component Z such as copper, bismuth, etc. is not always necessary, it has an improving effect on the activity and selectivity and is preferably used. When the amount of the component Z is too large, its effect hits the ceiling.

Materials and a method for preparing the catalyst are not limited to the materials and method described above. Any method may be employed so long as it finally gives the heteropolyacid structure described above.

Although the isobutane concentration in a starting gas to be subjected to reaction is no critical, it is preferably about 10 to about 70% by mole.

The molar ratio of molecular oxygen to isobutane is not critical. It is preferably about 0.1 -about 3.0, more preferably 0.2 - 1.0.

As a source of molecular oxygen, there may usually be used air, pure oxygen, oxygen-enriched air, etc.

It is preferable to incorporate water vapor into the starting gas for reaction in an amount of about 10 to about 40% by mole. Water vapor not only serves as a diluent for avoiding the range of explosion and removing the heat of reaction but also brings about a beneficial effect on the activity of reaction, the selectivity and the catalytic lifetime.

The starting gas for reaction may contain nitrogen, carbon dioxide, carbon monoxide, etc. When isobutylene is contained in the starting gas, it is converted into methacrylic acid and methacrolein, like isobutane.

The unreacted isobutane can be recovered and recycled. Methacrolein can be converted into methacrylic acid by recovery and recycling. When pure oxygen or oxygen-enriched air is used, it is preferable to recover and reuse the unreacted oxygen.

Although the reaction temperature can be chosen usually in the range of about 250° to about 350°C, it is preferably 280° to 330°C. At too low a temperature, the reaction rate is slow. At too high a temperature, the reaction selectivity is deteriorated and moreover the catalytic lifetime is shortened.

Although the reaction pressure can be chosen in the wide range of under reduced pressure to under pressure, it is industrially preferably atmospheric pressure to 3 atm.

The contact time between the reaction gas and the catalyst (a value obtained by dividing the volume of the catalyst by the flow rate of the reaction gas converted to a flow rate attained at a standard temperature and a standard pressure) is preferably about 1 to about 10 seconds.

3

As to the type of reactor, any reactor may freely be selected from static beds, fluidized beds, moving beds, etc., though a shell-and-tube static bed is preferably used.

Main products of the reaction other than methacrylic acid and methacrolein are carbon monoxide, carbon dioxide and acetic acid. All of them are useful components, and they can be separated and purified by conventional means such as cooling, absorption, extraction, distillation, etc. and taken out as products. The heat of reaction is recovered in the form of steam or the like and used as an energy source.

According to the process of the present invention, methacrylic acid and methacrolein can be directly obtained with high selectivity at a lower temperature than before by using a high concentration of isobutane. Furthermore, the catalytic lifetime is greatly improved and methacrylic acid and methacrolein can be efficiently produced.

The following examples serve to give specific illustration of the present invention but they are not intended in any way to limit the scope of the present invention.

The conversion of isobutane ($C_{IB}$), the selectivity for methacrylic acid ($S_{MAA}$) and the selectivity for methacrolein ($S_{MAR}$) are defined as follows:

$$C_{IB}\ (\%) = \frac{\text{Number of moles of isobutane reacted}}{\text{Number of moles of isobutane fed}} \times 100$$

$$S_{MAA}\ (\%) = \frac{\text{Number of moles of methacrylic acid produced}}{\text{Number of moles of isobutane reacted}} \times 100$$

$$S_{MAR}\ (\%) = \frac{\text{Number of moles of methacrolein produced}}{\text{Number of moles of isobutane reacted}} \times 100$$

Example 1

To a solution of 236 g of phosphomolybdic acid ($H_3P_1Mo_{12}O_{40} \cdot 30H_2O$) and 2.3 g of 85% phosphoric acid ($H_3PO_4$) in 400 ml of water ($H_2O$) was added 9.1 g of vanadium pentaoxide ($V_2O_5$), and the resulting mixture was refluxed with heating for about 6 hours to obtain an orange-colored solution. This solution is named solution A. A solution of 20 g of ammonium nitrate ($NH_4NO_3$) and 29.2 g of cesium nitrate ($CsNO_3$) in 200 ml of water (this solution is named solution B) was add to solution A, and the mixture thus obtained was evaporated to dryness as it was with a rotary evaporator and then calcined in a nitrogen ($N_2$) stream at 430°C for 5 hours.

The dark-blue powder thus obtained was molded into cylindrical tablets having a diameter of 5 mm and a height of 5 mm, whereby a catalyst was obtained.

The composition of the catalyst determined for elements other than oxygen, nitrogen and hydrogen was $Mo_{12}P_{1.2}V_1Cs_{1.5}$. In X-ray diffraction, the catalyst showed peaks at $2\theta(CuK\alpha) = 26°, 10.5°, 30°, 18.2$, etc. Thus, it had a heteropolyacid salt structure.

A Pyrex glass reaction tube having an inside diameter of 15 mm was packed with 6 ml of the catalyst. Then, to the tube was fed a starting gas containing 26% by mole of isobutane, 62% by mole of air and 12% by mole of water vapor, so as to adjust the contact time to 3.6 seconds (in terms of a contact time at standard state). The reaction pressure was adjusted to 1.5 atm and the temperature of wall of the reactor to 320°C. After 15 hours, the reaction product gas was analyzed by a gas chromatography to find the following; the conversion of isobutane: 9.5%, the selectivity for methacrylic acid: 48.5%, and the selectivity for methacrolein: 12.9%.

Example 2

4

A catalyst having the composition, $Mo_{12}P_{1.2}V_1Cs_{1.5}Cu_{0.2}$ was prepared in the same manner as in Example 1, except that a solution of 29.2 g of cesium nitrate and 4.8 g of copper nitrate ($Cu(NO_3)_2 \cdot 3H_2O$) in 200 ml of water was used in place of solution B, and that the calcination conditions were changed to 380°C for 5 hours in air. In X-ray diffraction, this catalyst showed peaks due to a heteropolyacid salt at $2\theta$-(CuK$\alpha$) = 26°, 10.5°, 30°, 18.2°, etc. and peaks due to free heteropolyacid at $2\theta$(CuK$\alpha$) = 8°, 8.4°, 9°, etc. Thus, the catalyst was a mixture of the heteropolyacid and the salt thereof. The catalyst was not in reduced state and has an orange color.

Using said catalyst, the reaction was carried out under the same reaction conditions as in Example 1 to obtain the following results; the conversion of isobutane: 8.8%, the selectivity for methacrylic acid: 51.2%, and the selectivity for methacrolein: 15.2%.

Example 3

The molded catalyst obtained in Example 1 was calcined in air at 380°C for another 5 hours. The calcination changed the color of the catalyst from dark blue to green. The green color indicates that the calcined product thus obtained was in a highly oxidized state.

Using this calcined product, the reaction was carried out under the same reaction conditions as in Example 1 to obtain the following results; the conversion of isobutane: 9.0%, the selectivity for methacyric acid: 47.3%, and the selectivity for methacrolein: 16.3%.

Comparative Example 1

A catalyst having the Composition, $Mo_{12}P_1$ was prepared by molding ammonium phosphomolybdate powder into cylindrical tablets having a diameter of 5 mm and a height of 5 mm and calcining the tablets at 430°C for 5 hours in a nitrogen stream.

Using this catalyst, the reaction was carried out under the same reaction conditions as in Example 1 to obtain the following results; the conversion of isobutane: 3.6%, the selectivity for methacrylic acid: 34.4%, and the selectivity for methacrolein: 20.8%.

Comparative Example 2

A catalyst having the composition, $Mo_{12}P_{1.2}V_1$ was prepared in the same manner as in Example 1, except that a solution of 20 g of ammonium nitrate in 100 ml of water was used in place of solution B.

Using this catalyst, the reaction was carried out under the same reaction conditions as in Example 1 to obtain the following results; the conversion of isobutane: 3.3%, the selectivity for methacrylic acid: 30.8%, and the selectivity for methacrolein: 30.4%.

Comparative Example 3

A catalyst having the composition, $Mo_{12}P_1Cs_{1.5}$ was prepared in the same manner as in Example 2, except that a solution of 236 g of phosphomolybdic acid in 400 ml of water was used in place of solution A.

Using this catalyst, the reaction was carried out under the same reaction conditions as in Example 1 to obtain the following results; the conversion of isobutane: 10.3%, the selectivity for methacrylic acid: 31.4%, and the selectivity for methacrolein: 6.9%.

Example 4

A catalyst having the composition, $Mo_{12}P_{1.2}V_1Rb_{1.5}$ Was prepared in the same manner as in Example 1, except for using 22 g of rubidium nitrate ($RbNO_3$) in place of cesium nitrate.

Using this catalyst, the reaction was carried out under the same reaction conditions as in Example 1 to obtain the following results; the conversion of isobutane: 8.0%, the selectivity for methacrylic acid: 45.8%, and the selectivity for methacrolein: 15.3%.

Example 5

A catalyst having the composition, $Mo_{12}P_{1.2}V_1Tl_{1.5}$ was prepared in the same manner as in Example 1, except for using 40 g of thallium nitrate ($TlNO_3$) in place of cesium nitrate.

Using this catalyst, the reaction was carried out under the same reaction conditions as in Example 1 to obtain the following results; the conversion of isobutane: 11.3%, the selectivity for methacrylic acid: 42.1%, and the selectivity for methacrolein: 11.5%.

Example 6

A catalyst having the composition, $Mo_{12}P_1As_{0.4}Cs_{2.0}Cu_{0.1}$ was prepared in the same manner as in Example 1, except that a solution obtained by adding 39.0 g of cesium nitrate, 2.4 g of copper nitrate and 20 g of ammonium nitrate in 200 ml of water to a solution of 236 g of phosphomolybdic acid and 9.5 g of a 60% aqueous arsenic acid ($H_3AsO_4$) solution in 400 ml of water was used.

Using this catalyst, the reaction was carried out under the same reaction conditions as in Example 1 to obtain the following results; the conversion of isobutane: 12.1%, the selectivity for methacrylic acid: 51.0%, and the selectivity for methacrolein: 10.2%.

Example 7

A catalyst having the composition, $Mo_{12}P_1As_{0.4}V_{0.5}Cs_{1.5}$ was prepared in the same manner as in Example 1, except for using 9.5 g of an aqueous arsenic acid solution as a material for solution A in place of phosphoric acid, and changing the amount of vanadium pentaoxide, another material for solution A, to 4.6 g.

Using this catalyst, the reaction was carried out under the same reaction conditions as in Example 1 to obtain the following results; the conversion of isobutane: 8.6%, the selectivity for methacrylic acid: 42.1%, and the selectivity for methacrolein: 22.5%.

Example 8

A catalyst having the composition, $Mo_{12}O_{1.2}V_1Cs_{1.5}Ag_{0.2}$ was obtained in the same manner as in Example 2, except that a solution of 29.2 g of cesium nitrate and 3.4 g of silver nitrate ($AgNO_3$) in 200 ml of water was used in place of solution B.

Using this catalyst, the reaction was carried out under the same reaction conditions as in Example 1 to obtain the following results; the conversion of isobutane: 9.2%, the selectivity for methacrylic acid: 46.3%, and the selectivity for methacrolein: 18.4%.

Example 9

A catalyst having the composition, $Mo_{12}P_{1.2}V_1Cs_{1.5}Bi_{0.4}$ was obtained in the same manner as in Example 2, except that a solution of 29.2 g of cesium nitrate and 19.4 g of bismuth nitrate ($Bi(NO_3)_3 \cdot 5H_2O$) in a 5% aqueous nitric acid ($HNO_3$) solution was used in place of solution B.

Using this catalyst, the reaction was carried out under the same reaction conditions as in Example 1 to obtain the following results; the conversion of isobutane: 8.4%, the selectivity for methacrylic acid: 43.2%, and the selectivity for methacrolein: 19.8%.

Comparative Example 4

A catalyst was prepared in accordance with Example 24 of JP-A-63-145249.

To a solution of 48.5 g of bismuth nitrate in 500 ml of a 5% aqueous nitric acid solution was add 236 g of phosphomolybdic acid and then 52.8 g of ascorbic acid, and stirred at 60°C for 1 hour. 202 Grams of finely powdered silica (Aerosil-400, Nippon Aerosil, Inc.) was added thereto and stirred for 1 hour. The resulting mixture was dried with a rotary evaporator and the solid thus obtained was calcined in air at

6

350°C for 1 hour to obtain a catalyst having the composition of $Mo_{12}P_1Bi_1Si_{33.6}$.

Using this catalyst, the reaction was carried out under the same reaction conditions as in Example 1 to obtain the following results; the conversion of isobutane: 5.1%, the selectivity for methacrylic acid: 8.6%, and the selectivity for methacrolein: 2.1%.

Example 10

A catalyst having the composition, $Mo_{12}P_{1.2}V_1Cs_{1.5}Co_{0.4}$ was obtained in the same manner as in Example 8, except for using 11.6 g of cobalt nitrate ($Co(NO_3)_2 \cdot 6H_2O$) in place of silver nitrate.

Using this catalyst, the reaction was carried out under the same reaction conditions as in Example 1 to obtain the following results; the conversion of isobutane: 8.6%, the selectivity for methacrylic acid: 45.2%, and the selectivity for methacrolein: 16.2%.

Example 11

A catalyst having the composition, $Mo_{12}P_{1.2}V_1Cs_{1.5}Fa_{0.4}$ was obtained in the same manner as in Example 8, except for using 16.2 g of iron nitrate ($Fe(NO_3)_2 \cdot 9H_2O$) in place of silver nitrate.

Using this catalyst, the reaction was carried out under the same reaction conditions as in Example 1 to obtain the following results; the conversion of isobutane: 8.8%, the selectivity for methacrylic acid: 46.3%, and the selectivity for methacrolein: 15.2%.

Example 12

A catalyst having the composition, $Mo_{12}P_{1.2}V_1Cs_{1.5}La_{0.4}$ was obtained in the same manner as in Example 8, except for using 17.3 g of lanthanum nitrate ($La(NO_3)_3 \cdot 6H_2O$) in place of silver nitrate.

Using this catalyst, the reaction was carried out under the same reaction conditions as in Example 1 to obtain the following results; the conversion of isobutane: 9.3%, the selectivity for methacrylic acid: 44.4%, and the selectivity for methacrolein: 13.3%.

Example 13

A catalyst having the composition, $Mo_{12}P_{1.2}V_1Cs_{1.5}Ce_{0.4}$ was obtained in the same manner as in Example 8, except for using 17.4 9 of cerium nitrate ($Ce(NO_3)_3 \cdot 6H_2O$) in place of silver nitrate.

Using this catalyst, the reaction was carried out under the same reaction conditions as in Example 1 to obtain the following results; the conversion of isobutane: 9.2%, the selectivity for methacrylic acid: 47.9%, and the selectivity for methacrolein: 12.6%.

Example 14

A catalyst having the composition, $Mo_{12}P_{1.2}V_1Cs_{1.5}Sb_1$ was obtained in the same manner as in Example 2, except that a suspension prepared by dissolving 29.2g of cesium nitrate in 200 ml of water and suspending 29.1 g of antimony trioxide ($Sb_2O_3$) in the resulting solution was used in place of solution B.

Using this catalyst, the reaction was carried out under the same reaction conditions as in Example 1 to obtain the following results; the conversion of isobutane: 8.4%, the selectivity for methacrylic acid: 43.7%, and the selectivity for methacrolein: 17.1%.

Comparative Example 5

A catalyst was prepared in accordance with Example 1 of JP-A-55-62041.

To a solution of 17.8 g of 70% nitric acid and 24 g of a 30% aqueous ammonia solution in 800 ml of water was added dropwise 15.0 g of antimony pentachloride ($SbCl_5$), and 118 g of phosphomolybdic acid was added to the resulting slurry and stirred with heating for 16 hours. The mixture thus obtained was concentrated and dried with a rotary evaporator and then calcined in air at 400°C for 6 hours. The calcined product was molded into tablets to obtain a catalyst having the composition of $Mo_{12}P_1Sb_1$.

Using this catalyst, the reaction was carried out under the same reaction conditions as in Example 1 to obtain the following results; the conversion of isobutane: 4.8%, the selectivity for methacrylic acid: 29.6%, and the selectivity for methacrolein: 16.8%.

Example 15

To 600 ml of water were added 7.5 g of 85% phosphoric acid and 4.7 g of 60% arsenic acid and then 105.9 g of ammonium molybdate (($NH_4$)$_6$$Mo_7O_{24}$•$4H_2O$), 2.28 g of vanadium pentaoxide, 2.17 g of copper phosphate ($Cu_3(PO_4)_2$•$3H_2O$) and 35 g of cesium nitrate. The resulting slurry was concentrated and dried with a rotary evaporator after refluxing at 100°C for 15 hours, and then calcined at 430°C for 5 hours in a nitrogen stream. The calcined product was molded to obtain a catalyst having the composition of $Mo_{12}P_{1.5}V_{0.5}As_{0.4}Cs_{1.8}Cu_{0.3}$.

Using this catalyst, the reaction was carried out under the same reaction conditions as in Example 1 to obtain the following results; the conversion of isobutane: 11.7%, the selectivity for methacrylic acid: 53.1%, and the selectivity for methacrolein: 10.4%.

In addition, the reaction was continued for 100 hours under the same reaction conditions. The results were as follows; the conversion of isobutane: 10.5%, the selectivity for methacrylic acid: 55.1%, and the selectivity for methacrolein: 15.8%.

Furthermore, the reaction was further continued for 1,000 hours under the same reaction conditions. The results were as follows; the conversion of isobutane: 10.2%, the selectivity for methacrylic acid: 55.0%, and the selectivity for methacrolein: 16.3%.

Example 16

The catalyst obtained in Example 15 was used. A Pyrex glass reaction tube having an inside diameter of 15 mm was packed with said catalyst and thereto was fed a starting gas containing 15% by mole of isobutane, 35% by mole of oxygen, 35% by mole of water vapor and 15% by mole of nitrogen, so as to adjust the contact time to 1.8 seconds (in terms of a contact time at standard state). The reaction pressure was adjusted to 1.5 atm and the temperature of the wall of the reactor to 320°C.

After 15 hours, the reaction product gas was analyzed by a gas chromatography to find the following; the conversion of isobutane: 10.8%, the selectivity for methacrylic acid: 52.9%, and the selectivity for methacrolein: 12.8%.

Example 17

The catalyst obtained in Example 15 was used. A Pyrex glass reaction tube having an inside diameter of 15 mm was packed with said catalyst. Through the reaction tube, a mixed gas of 62% by mole of isobutane and 38% by mole of water vapor was passed so as to adjust the contact time to 3.6 seconds, for 5 seconds, and a mixed gas of 62% by mole of air and 38% by mole of water vapor was passed so as to adjust the contact time to 3.6 seconds, for 10 seconds. The two patterns of passage of these mixed gas was repeated with being interlocked with a timer. The reaction pressure was 1.0 atm and the reaction temperature 320°C.

After 15 hours, the reaction product gas was analyzed by a gas chromatography to find the following; the conversion of isobutane: 11.2%, the selectivity for methacrylic acid: 53.6%, and the selectivity for methacrolein: 11.5%.

Example 18

The catalyst obtained in Example 15 was used. A Pyrex glass reaction tube having an inside diameter of 15 mm was packed with 6 ml of said catalyst thereto was a starting gas containing 45% by mole of isobutane, 33% by mole of oxygen, 12% by mole of water vapor and 10% by mole of nitrogen, so as to adjust the contact time to 5 seconds (in terms of a contact time at standard state). The reaction pressure was adjusted to 1.5 atm and the temperature of the wall of the reactor to 300°C. After 15 hours, the reaction product gas was analyzed by a gas chromatography to find the following; the conversion of isobutane: 8.8%, the selectivity for methacrylic acid: 51.5%, and the selectivity for methacrolein: 13.3%.

## Claims

1. A process for producing methacrylic acid and methacrolein which comprises the step of:
subjecting isobutane to gas phase catalytic oxidation with molecular oxygen by the use of a catalyst comprising either a heteropolyacid salt or a mixture of a heteropolyacid and a salt thereof, said catalyst having the following composition determined for elements other than oxygen, nitrogen and hydrogen,
$P_a Mo_b X_c Y_d Z_e$
wherein P and Mo are phosphorus and molybdenum, respectively,
X is at least one member selected from the group consisting of rubidium, cesium and thallium,
Y is at least one member selected from the group consisting of vanadium and arsenic,
Z is at least one member selected from the group consisting of copper, silver, bismuth, iron, cobalt, antimony, lanthanum and cerium,
the suffixes a, b, c, d and e indicate the atomic ratio between the elements, and
a : b : c : d : e is at most 3 : 12 : at most 3 : at most 3 : O-3.

2. The process of claim 1, wherein e = 0.

3. The process of claim 1, wherein e = at most 3.

4. The process of claim 2, wherein
X is one member selected from the group consisting of rubidium, cesium and thallium, and
Y is at least one member selected from the group consisting of vanadium and arsenic.

5. The process of claim 2, wherein
X is one member selected from the group consisting of rubidium, cesium and thallium, and
Y is vanadium and arsenic.

6. The process of claim 2, wherein a : b : c : d = 1-1.2 : 12 : 0.4-1 : 1.5-2.

7. The process of claim 3, wherein
X is one member selected from the group consisting of rubidium, cesium and thallium,
Y is at least one member selected from the group consisting of vanadium and arsenic, and
Z is one member selected from the group consisting of copper, silver, bismuth, iron, cobalt, antimony, lanthanum and cerium.

8. The process of claim 3, wherein
X is one member selected from the group consisting of rubidium, cesium and thallium,
Y is vanadium and arsenic, and
Z is one member selected from the group consisting of copper, silver, bismuth, iron, cobalt, antimony, lanthanum and cerium.

9. the process of claim 3, wherein a : b : c : d : e = 1-1.2 : 12 : 0.4-1 : 1.5-2 : 0.1-0.4.

10. The process of claim 1, wherein the step includes selecting as the isobutane source a starting gas containing about 10 to about 70% by mole of isobutane.

11. The process of claim 1, wherein the step includes selecting a molar ratio of molecular oxygen to isobutane of about 0.1 : 1 to about 3.0 : 1.

12. The process of claim 1, wherein the step includes selecting a molar ratio of molecular oxygen to isobutane of about 0.2 : 1 to 1.0 : 1.

13. The process of claim 1, wherein the step includes selecting either air, pure oxygen or oxygen-enriched air as the molecular oxygen source.

14. The process of claim 1, wherein the step includes selecting as the isobutane source a starting gas containing about 10 to about 70% by mole of isobutane and about 10 to about 40% by mole of water vapor.

15. The process of claim 1, wherein the step includes selecting a reaction temperature of about 250° to about 350°C.

16. The process of claim 1, wherein the step includes selecting a reaction temperature of 280°C to 330°C.

17. The process of claim 1, wherein the step includes selecting a reaction pressure of atmospheric pressure to 3 atm.

18. The process of claim 1, wherein the step includes selecting a contact time of about 1 to about 10 seconds.

19. The process of claim 1, wherein the step includes selecting a shell-and-tube static bed as a reactor.

20. A process for producing acrylic acid, which comprises the steps of:

subjecting a starting gas containing isobutane to gas phase catalytic oxidation by bringing the same together with molecular oxygen into contact with a catalyst comprising either a heteropolyacid salt or a mixture of a heteropolyacid and a salt thereof, said catalyst having the following composition determined for elements other than oxygen, nitrogen and hydrogen,

$P_aMo_bX_cY_dZ_e$

wherein P and Mo are phosphorus and molybdenum, respectively,

X is at least one member selected from the group consisting of rubidium, cesium and thallium,

Y is at lest one member selected from the group consisting of vanadium and arsenic,

Z is at least one member selected from the group consisting of copper, silver, bismuth, iron, cobalt, antimony, lanthanum and cerium,

the suffixes a, b, c, d and e indicate the atomic ratio between the elements, and

a : b : c : d : e = at most 3 : 12 : at most 3 : at most 3 : 0-3, and

recycling the unreacted isobutane and methacrolein in admixture with the starting gas.